# EUROPEAN PATENT SPECIFICATION

(11) **EP 3 872 028 B1**
(45) Date of publication and mention of the grant of the patent: **31.01.2024**
(21) Application number: 19903690.6
(22) Date of filing: 11.12.2019
(51) Int. Cl.: B67D 1/07, B67D 1/08, G05B 23/02, G16Y 40/20, G16Y 10/45, B08B 9/032

(54) **SYSTEM AND METHOD FOR PREDICTING DIRT IN DRINKING FLUID PIPE**
SYSTEM UND VERFAHREN ZUR VORHERSAGE VON SCHMUTZ IN EINEM TRINKWASSERROHR
SYSTÈME ET PROCÉDÉ DE PRÉDICTION DE SALETÉ DANS UN TUYAU DE LIQUIDE POTABLE

(30) Priority: 28.12.2018 JP 2018246723
(43) Date of publication of application: 01.09.2021
(73) Proprietor: Asahi Group Holdings, Ltd., Tokyo 130-8602 (JP); Asahi Breweries, Ltd., Tokyo 130-8602 (JP)
(72) Inventor: KURABE, Yasuhiro, Ibaraki 302-0106 (JP); SUZUKI, Toru, Ibaraki 302-0106 (JP); YAMASHITA, Naoyuki, Ibaraki 302-0106 (JP); KITANO, Junichi, Ibaraki 302-0106 (JP); WADA, Takashi, Hyogo 650-0022 (JP); KUSUNOKI, Kenji, Hyogo 650-0022 (JP); NISHIO, Takashi, Hyogo 650-0022 (JP)
(74) Representative: Müller-Boré & Partner Patentanwälte PartG mbB
(86) International application number: PCT/JP2019/048433
(87) International publication number: WO 2020/137541

(56) References cited:
- JP-A- H10 302 141
- JP-A- 2003 192 096
- JP-A- 2011 092 316
- JP-A- 2016 085 572
- JP-A- 2018 173 408
- US-A1- 2010 072 225
- US-A1- 2014 166 053
- US-A1- 2017 088 410

## Description

### TECHNICAL FIELD

The present invention relates to a system and method for predicting dirt or contamination in a drinking fluid pipe, and more particularly to a system and method for predicting dirt or contamination in a pipe through which a drinking fluid such as beer flows.

### BACKGROUND ART

A liquid supply system is commonly used as a device for serving a liquid, for example, a beverage such as beer, in a restaurant and the like. With beer taken as an example, the liquid supply system includes a carbon dioxide cylinder, a beer barrel filled with beer, a supply pipe, and a beer dispenser, and applies pressure to the beer in the beer barrel with carbon dioxide in the carbon dioxide cylinder to forcibly feed the beer to the beer dispenser through the supply pipe. The beer dispenser includes a beer cooling pipe installed in a cooling tank, a freezer, and a spout, and works such that a part of cooling water in the cooling tank is frozen by the freezer, and the beer is caused to flow, by operation of a lever at the spout, through the beer cooling pipe while being cooled to pour into a beverage container such as a mug. As described above, the beer in the beer barrel is served to a customer.

On the other hand, the liquid supply system includes, as described above, a relatively long pipeline extending from an outlet of the beer barrel to the spout including the beer cooling pipe through which the beer flows, and the inside of the pipeline becomes dirty with use. Such dirt or contaminant causes various germs to propagate, leading to provision of beer that is not tasty due to generation of an odd taste or the like.

Therefore, from the viewpoint of hygiene control and quality control of beer to be served, a brewer instructs a user of the liquid supply system (for example, a restaurant) to regularly wash or clean the pipeline extending from the outlet of the beer barrel to the spout, for example, everyday after business hours to control hygiene and guarantee the quality of the beer to be served. Examples of such a washing or cleaning work include water washing with running water (tap water), for example, everyday after business hours, and washing with a designated sponge and washing water in which the designated sponge is moved in the pipeline while the washing water is caused to flow through the pipeline (hereinafter referred to as "sponge washing"), for example, once a week.

### PRIOR ART DOCUMENTS

### PATENT DOCUMENTS

Patent Document 1: JP 5393389 B
US 2014/166053 A1 relates to cleaning a drinking fluid pipe as portion of a beverage line of a beverage distribution system based upon a signal received from a remote source. A management server can manage a cleaning system from a remote location in which a remote signal can drive a cleaning system and at least one or more electric valves within the beverage distribution system.

However, US 2014/166053 A1 does not disclose that the beverage distribution system is configured to communicate information on temperature of the beverage in a storage container, and that the management server is configured to output a prediction about contamination in the drinking fluid pipe based on the information on whether or not washing work has been conducted, the information on temperature, and information on the liquid type of the beverage, wherein the prediction about contamination in a drinking fluid pipe is a number of days until a contamination level of the drinking fluid pipe exceeds a threshold level or the prediction is a ratio of a current contamination level to a threshold level (Lth).

US 2017/088410 A1 describes that among other things, beverages are dispensed from one or more beverage dispensers based on selections made by users. Information about the dispensing of the beverages is sent to a central server where it is used to manage a variety of functions including replacement of depleted supplies of components.

### SUMMARY OF THE INVENTION

### PROBLEMS TO BE SOLVED BY THE INVENTION

As described above, dirt or contamination in the drinking fluid pipe extending from the outlet of the beer barrel to the spout in the liquid supply system is an important matter for hygiene control and quality control of the beer to be served. At present, a contamination check is just a method in which a worker such as a brewer's sales representative visually compares turbidity of collected washing water with reference turbidity once every few months.

Note that the above-described Patent Document 1 discloses a description for determining whether or not, with the pipeline drained, washing work has been conducted and does not disclose a description for predicting contamination in the pipeline.

The present invention has been made to solve the above-described problem, and it is therefore an object of the present invention to provide a system and method for predicting contamination in a drinking fluid pipe, allowing contamination management in the drinking fluid pipe in a liquid supply system to be at a level higher than in the related art.

### MEANS FOR SOLVING THE PROBLEMS

In order to achieve the above-described object, the present invention is configured as defined in claim 1 and claim 6.

### EFFECTS OF THE INVENTION

In the system for predicting contamination in the drinking fluid pipe, the management server is configured to output the prediction about contamination in the drinking fluid pipe extending from the outlet of the storage container to the spout in the liquid supply system based on the information on whether or not washing work has been conducted, the information on temperature of the beverage in the storage container, and the information on liquid type of the beverage. This allows dirt or contamination in the drinking fluid pipe to be controlled or managed at a level higher than in the related art.

### BRIEF DESCRIPTION OF THE DRAWINGS

Fig. 1 is a block diagram showing an example of a structure of a system for predicting contamination according to an embodiment.
Fig. 2 is a diagram showing an example of a structure of a liquid supply system shown in Fig. 1.
Fig. 3A is a perspective view of a washing degree determination device shown in Fig. 1.
Fig. 3B is a cross-sectional view of a sampling container provided in the washing degree determination device shown in Fig. 3A.
Fig. 3C is a block diagram schematically showing a structure of the washing degree determination device shown in Fig. 3A.
Fig. 3D is a schematic cross-sectional view of the sampling container shown in Fig. 3B set in a holder of the washing degree determination device shown in Fig. 3A.
Fig. 3E is a flowchart for describing a measurement operation in the washing degree determination device shown in Fig. 3A.
Fig. 4A is a block diagram showing an example of a structure of a management server shown in Fig. 1.
Fig. 4B is a diagram showing an example of a hardware structure of the management server shown in Fig. 4A.
Fig. 4C is a diagram showing an example of a structure of a learned model stored in the management server shown in Fig. 4A.
Fig. 4D is a flowchart showing an example of learning processing performed by a learning processor provided in the management server shown in Fig. 4A.
Fig. 4E is a flowchart for describing a contamination prediction operation performed by the management server shown in Fig. 4A.
Fig. 4F is a diagram for describing changes in contamination level related to construction of an original teacher model shown in Fig. 4A.

### EMBODIMENTS OF THE INVENTION

A description will be given of a system and method for predicting dirt or contamination in a drinking fluid pipe according to the embodiment of the present invention with reference to the drawings. In the drawings, the same or similar components are denoted by the same reference numerals. Further, in order to avoid the following description being unnecessarily redundant and to help those skilled in the art to easily understand the following description, detailed description of already well-known matters and redundant description of substantially the same configuration may be omitted. Further, the following description and the accompanying drawings are not intended to limit the subject matter of the claims.

The system for predicting contamination in a drinking fluid pipe according to the present embodiment is configured to predict contamination in a drinking fluid pipe in a liquid supply system to be described below as an example and outputs the prediction about the contamination.

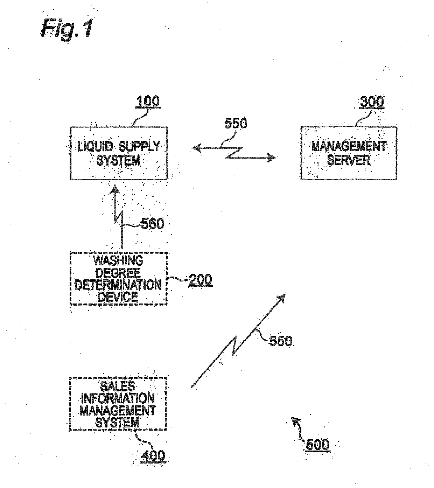

As shown in Fig. 1, such a system 500 for predicting contamination in the drinking fluid pipe includes a liquid supply system 100 and a management server 300, and may further include a washing degree determination device 200 and a sales information management system 400.

The outlines of such components will be described. The liquid supply system 100 and the sales information management system 400 are installed in each restaurant or the like. The sales information management system 400 is configured to manage sales information and is equivalent to a so-called point of sales system (POS) that is already available. The POS system is configured to read or obtain and transmit sales information and the like upon, for example, payment made by a customer. The management server 300 is, for example, a computer connected to a communication line 550 and is equivalent to so-called cloud computing, and the management server 300 is managed by, for example, a brewer. The washing degree determination device 200 is a measuring instrument that can be carried by a worker such as a sales representative of the brewer and is capable of measuring a washing degree of the drinking fluid pipe in the liquid supply system 100.

The liquid supply system 100 and the sales information management system 400 are each connected to the management server 300 over the communication line 550, and the washing degree determination device 200 is connected to the liquid supply system 100 by using, for example, a near-field radio communication function 560.

Each component will be described in detail below one by one.

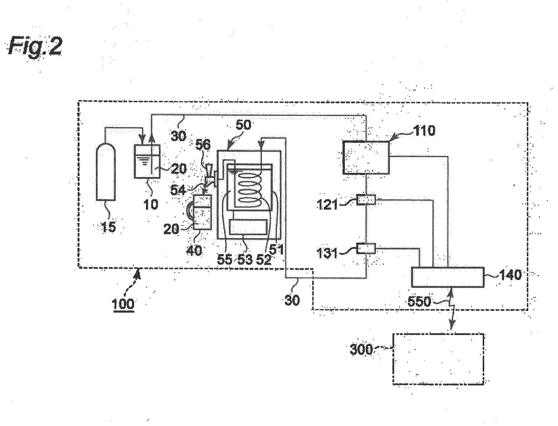

First, a description will be given of the liquid supply system 100 with reference to Fig. 2. In the following description, beer is taken as an example of a beverage handled by the liquid supply system 100, but the liquid supply system 100 can handle not only beer, but also alcoholic beverages such as low-malt beer, liqueur, Japanese spirit with soda, i.e. shochu highball, whiskey, and wine, drinking water, soft drinks, carbonated drinks, and the like.

The liquid supply system 100 includes a storage container 10, a pressurizing source 15, a supply pipe 30, a dispensing device 50, a washing mode detector 110, a temperature sensor 121, and a communication unit 140, and may further include a liquid type sensor 131.

In such a liquid supply system 100, a beverage (for example, beer) 20 in the storage container 10 is supplied or forcibly fed to the dispensing device 50 through the supply pipe 30 under pressure applied by the pressurizing source 15 to pour from the dispensing device 50 into a beverage container (for example, a mug) 40. Herein, the storage container 10 is, for example, a container made of stainless steel, a so-called beer barrel filled with beer by a brewer, and has a capacity of, for example, 5 liters, 10 liters, 19 liters, or the like. The pressurizing source 15 is a carbon dioxide cylinder. The supply pipe 30 is a flexible tube made of resin such as polyamide, polyurethane, or polyester, which allows beer to flow from the storage container 10 to the dispensing device 50. It is preferable that a fluid pipeline including the supply pipe 30 and extending to a liquid spout or a liquid dispensing outlet 54 of the dispensing device 50 be designed to have a uniform inner diameter to facilitate sponge washing, i.e. a cleaning by using a sponge, in the fluid pipeline.

A description will be given of, as an example of the above-described dispensing device 50, a beer dispenser (sometimes referred to as a "beer server") according to the present embodiment (hence, hereinafter, the dispensing device 50 may be referred to as a beer dispenser 50). The beer dispenser 50 includes a liquid cooling pipe (a beer cooling pipe according to the embodiment) 52 installed in a cooling tank 51, a freezer, i.e. a refrigeration machine, 53, the liquid spout 54, and the like, the freezer 53 includes a compressor, a condenser, a refrigerant pipe installed in the cooling tank 51, and the like that are provided for a refrigerant, and a part of cooling water 55 in the cooling tank 51 is frozen by the freezer 53. In such a beer dispenser 50, the beer forced to fed by the pressurizing source 15 in response to operation of a lever 56 provided on the spout 54 flows through the beer cooling pipe 52 to be cooled by heat exchange with the cooling water 55 to pour into the beverage container 40 such as a mug, and is then served to a customer.

Note that the beer dispenser 50 is generally used in an environment where the outside air temperature is 5°C or more and 40°C or less. Further, according to the embodiment, the beer dispenser 50 cools beer that is the beverage of interest 20, but the dispensing device 50 according to the embodiment may heat or keep the beverage of interest 20 warm.

The washing mode detector 110 is a means for detecting that the liquid supply system 100 has shifted to a washing or cleaning mode, that is, the above-described water washing work.

Herein, the water washing work or cleaning is conducted by changing liquid flowing through the supply pipe 30 from beer to washing water and forcing the washing water to flow. Due to the water washing work after business hours, beer remaining in the drinking fluid pipe from the outlet of the storage container 10 to the spout 54 is drained from the spout 54 while being replaced with the washing water.

After the water washing is conducted as described above, as a preparation to serve beer next business time, the washing water remaining in the drinking fluid pipe from the outlet of the storage container 10 to the spout 54 is replaced with carbon dioxide blown into the pipe, thereby making the drinking fluid pipe in an empty state. The water washing operation is conducted by such a series of operations. Therefore, at the end of the water washing work, the drinking fluid pipe extending from the storage container 10 to the spout 54 has been completely drained.

As described above, in the "washing mode", the supply pipe 30 changes in inner state thereof. Specifically, the inside of the supply pipe 30 changes roughly from beer to water and from water to gas according to the present embodiment. Therefore, the washing mode detector 110 may be any means capable of detecting such a state transition in the supply pipe 30. Alternatively, the washing mode detector 110 may use an empty-liquid detection sensor capable of detecting a liquid such as beer or water, or gas in the supply pipe 30 or at least detecting that the liquid has turned into gas. Examples of the empty-liquid detection sensor can be used with an optical sensor, a capacitance sensor, a conductivity sensor, and the like. Further, pressure applied to a pipe wall of the resin-made supply pipe 30 varies in a manner that depends on the state transition in the supply pipe 30; therefore, a pipe-pressure (pressure) sensor installed on an outer surface of the pipe wall may also be used.

Further, as the washing mode detector 110, the following device may be used instead of the various sensors described above.

That is, the applicant of the present application has been providing a fluid flow path adjustment device that can be installed in the liquid supply system 100, such as disclosed in JP 5649801 B. The fluid flow path adjustment device is disposed in the supply pipe 30 and is configured to prevent, when the beer in the storage container 10 runs out during pouring from the spout 54 to the beverage container 40 (i.e., the storage container 10 becomes empty) or when the storage container 10 is replaced, carbon dioxide that is a pressurized gas from being ejected from the liquid spout 54 of the dispensing device 50. In order to achieve this object, the fluid flow path adjustment device has a liquid state determination unit including a light emitting element and a light receiving element, for being used to determine a state of fluid flowing through the supply pipe 30. Therefore, this fluid flow path adjustment device may also be used as the washing mode detector 110.

Furthermore, the above-described fluid flow path adjustment device includes an on/off switch that is set to off by a store clerk after business hours. That is, as described above, from the viewpoint of hygiene and the like, it is necessary to stop the operation of the fluid flow path adjustment device for the water washing work in accordance with recommending the washing operation in the pipe after business hours. This makes it possible to determine that setting the on/off switch to off causes a shift to the water washing work. Accordingly, the fluid flow path adjustment device can be regarded as the washing mode detector 110. Even after the on/off switch is set to off, the inside of the fluid flow path adjustment device remains energized, and necessary functional parts are thus in a standby state.

The washing mode detector 110 described above is electrically connected to the communication unit 140 and is capable of sending information on whether or not the water washing work has been conducted to the management server 300 via the communication unit 140. The information thus sent can include not only the information on whether or not the washing work has been conducted, but also time information representing date and time when the washing work is conducted and elapsed time information representing a period from the previous washing work to the current washing work, which can be obtained by comparing time information on the previous washing work and time information on the current washing work.

Such information on whether or not the water washing work has been conducted is directly related to a prediction with respect to contamination in the drinking fluid pipe extending from the outlet of the storage container 10 to the spout 54 and is thus the most important factor.

The temperature sensor 121 is disposed at a suitable position between the storage container 10 and the dispensing device 50, preferably near the outlet of the storage container 10 and measures a liquid temperature of the beverage 20 flowing through the supply pipe 30, that is, a temperature of the beverage 20 (beer) stored in the storage container 10. Examples of such a temperature sensor 121 include a thermocouple, a resistance thermometer, a thermistor, and the like. Note that since the temperature sensor 121 measures the temperature of the beverage 20, it is needless to say that the temperature sensor 121 is installed in the supply pipe 30 with a structure that complies with prescribed regulations. A measured value detected by the temperature sensor 121 is sent to the communication unit 140.

The applicant obtains that there is a correlation between the liquid temperature of the beverage 20 and contamination in the drinking fluid pipe; therefore, measuring the liquid temperature of the beverage 20 flowing through within the supply pipe 30, that is, the temperature of the beverage 20 in the storage container 10, with the temperature sensor 121 is one of the important factors in predicting contamination in the drinking fluid pipe extending from the outlet of the storage container 10 to the spout 54 and is thus significant. That is, the storage container 10 is, in many cases, placed in an ambient temperature atmosphere, and the liquid temperature of the beverage 20 tends to be higher, especially in the summer. Accordingly, the liquid temperature of the beverage 20 is an important factor in predicting contamination in the pipe.

The liquid type sensor 131 is a sensor disposed at a suitable position between the storage container 10 and the dispensing device 50 to determine the liquid type of the beverage 20. Examples of such a liquid type sensor 131 include an optical sensor, a conductivity sensor, and the like. On the other hand, in a practical application, the liquid type of the beverage 20 flowing through in the dispensing device 50 does not change frequently, and the liquid type is basically fixed. Therefore, information on the liquid type is usually input to the management server 300. Therefore, the liquid type sensor 131 is not an indispensable sensor.

Note that the location of the liquid type sensor 131 is not limited to the position described above, and the liquid type sensor 131 may be set to the supply pipe 30 in the dispensing device 50, for example. Further, when the liquid type sensor 131 is installed, detection information obtained by the liquid type sensor 131 is sent to the communication unit 140.

The applicant obtains that there is a correlation between the type of the beverage 20 and contamination in the drinking fluid pipe. For example, beer, which is a brewed beverage, contains a beer extract, so that beer tends to cause contamination within the pipe as compared with distilled alcoholic beverages. Further, even beer causes a difference in generation of contamination and contamination level in a manner that depends on at least one of a type of ingredients or an amount of the ingredients, and depends on a difference in type of beer. Therefore, the liquid type of the beverage 20 flowing through within the supply pipe 30 is one of the important factors in predicting contamination in the drinking fluid pipe extending from the outlet of the storage container 10 to the spout 54, and is thus significant.

Further, as described above, since the fluid flow path adjustment device disclosed in, for example, JP 5649801 B is capable of determining a state of a fluid flowing through within the supply pipe 30, the fluid flow path adjustment device provided with the liquid state determination unit including a light emitting element and a light receiving element may be used as the liquid type sensor 131.

The communication unit 140 transmits each piece of information given from the washing mode detector 110, the temperature sensor 121, and the liquid type sensor 131 described above to the management server 300 via the communication line 550. The communication unit 140 further transmits store information on a store such as a restaurant (for example, information on a name, location, and the like of, for example, a restaurant) where the liquid supply system 100 is installed together with time information representing date and time, and the like. Further, the communication unit 140 may not only transmit information, but also receive information from the washing degree determination device 200 to be described later, and may transmit/receive information to/from the management server 300.

The liquid supply system 100 may further include a flow rate sensor that detects a flow (flow rate) of the beverage 20.

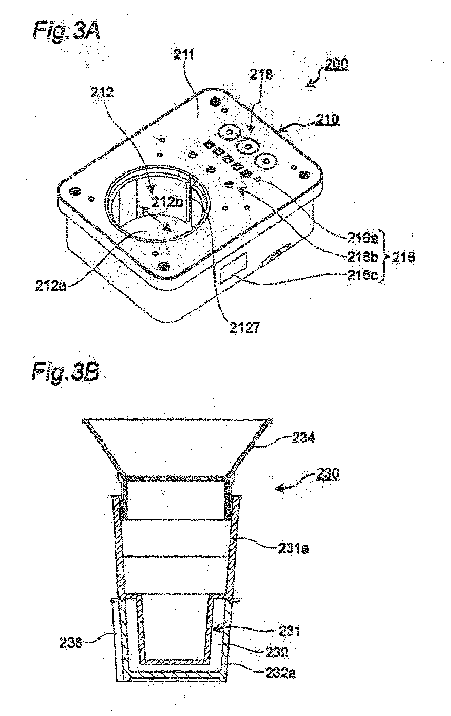

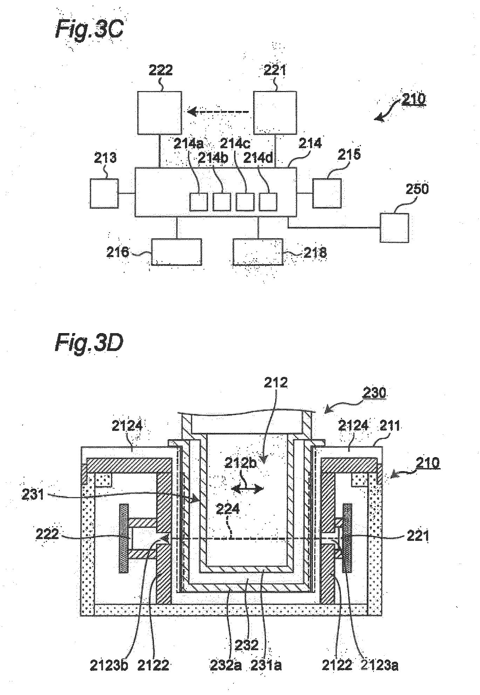

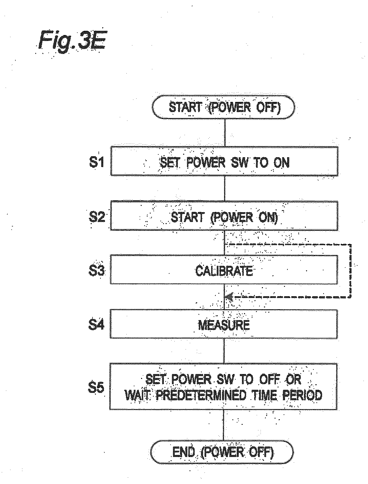

Next, a description will be given of the washing degree determination device 200 with reference to Figs. 3A to 3E (sometimes collectively referred to as "Fig. 3"). Note that it is necessary to measure turbidity made by the worker using the washing degree determination device 200 to transmit information on measurement result of the turbidity.

The washing degree determination device 200 according to the present embodiment uses water of the sponge washing (hereinafter referring to as "sponge washing water") as a liquid to be measured, which is collected from the liquid supply system 100 described above, to measure the turbidity of the washing water. Further, the device 200 determines quality, i.e. good or bad, of the washing work with respect to the liquid supply system 100 based on a measurement result of the turbidity, and supplies such pieces of information to the management server 300.

Such a washing degree determination device 200 has a configuration that the worker such as the sales representative of the brewer can carry with, and primarily includes a measuring instrument 210 shown in Fig. 3A, a sampling container 230 shown in Fig. 3B, and a transmitter 250 (Fig. 3C). The device 200 measures the turbidity of the liquid to be measured filled in the sampling container 230 set to a holder 212 of the measuring instrument 210 by using a light transmission technique, and further determines good or bad judgment of the washing work.

A description will be given below of the measuring instrument 210 and the sampling container 230.

As shown in Figs. 3A and 3C, the measuring instrument 210 includes the holder 212, a controller 214, a display 216, and the transmitter 250, further includes switches 218 provided on a front surface 211, and holds a battery serving as a power source 213 in a replaceable manner.

As shown in Figs. 3A and 3D, the holder 212 includes, for example, a recess 212a having a circular cup shape, holds the sampling container 230 in a detachable manner, and includes a light projecting element 221 and a light receiving element 222 arranged facing each other in a diametrical direction 212b of the recess 212a. The light projecting element 221 and the light receiving element 222 are configured to project and receive near infrared light and form an optical path 224 in the diametrical direction 212b.

Such a holder 212 is formed of a two-layer wall including an outer wall 2122 and an inner wall 2124, and the outer wall 2122 is formed of a light-shielding member made of resin such as black resin, and includes a light projecting side opening 2123a and a light receiving side opening 2123b on the optical axis of the optical path 224. In the outer wall 2122, mounting seats for the light projecting element 221 and the light receiving element 222 are provided corresponding to the light projecting side opening 2123a and the light receiving side opening 2123b, and a substrate on which the light projecting element 221 is mounted and a substrate on which the light receiving element 222 is mounted are each closely attached to a corresponding one of the mounting seats in a light-shielded state.

The inner wall 2124 is a light-transparent member that covers entire surface of the outer wall 2122 and is in contact with the sampling container 230. Therefore, light projected from the light projecting element 221 through the light projecting side opening 2123a passes through the inner wall 2124 and the light receiving side opening 2123b and then is arrived at the light receiving element 222. Further, provided in the inner wall 2124 is a holder-side positioning part 2127 that allows the sampling container 230 to be disposed at a fixed position and orientation relative to the holder 212.

A description will be given below of the sampling container 230 with reference to Fig. 3B.

The sampling container 230 is a cup-shaped container that is detachably held with respect to the holder 212 and is molded of a light-transparent material such as a resin material or a glass material that allows the light projecting element 221 to project light and allows the light receiving element 222 to receive light. Such a sampling container 230 includes an inner container 231a with a liquid storage portion 231 located at a lower part of the sampling container 230, the inner container 231a allowing a basket 234 to be detachably attached to an upper opening of the inner container 231a, and an outer container 232a with a size surrounding the liquid storage portion 231. The liquid storage portion 231 stores a predetermined amount (about 50 mL to 80 mL as an example) of the sponge washing water that is the liquid to be measured. When the sampling container 230 is mounted to the holder 212, the optical path 224 extends in the diametrical direction 212b through the liquid storage portion 231. Further, in order to prevent condensation on the liquid storage portion 231, the liquid storage portion 231 has a double wall structure where a periphery of the liquid storage portion 231 is sealed with the outer container 232a to provide an air layer 232. On the other hand, when the liquid to be measured stored in the liquid storage portion 231 has a liquid temperature that does not cause condensation, the sampling container 230 need not have the condensation prevention structure. Further, provided on a peripheral surface of the outer container 232a is a container-side positioning part 236 that is engageable with the holder-side positioning part 2127 of the holder 212, the container-side positioning part 236 projecting from the peripheral surface of the outer container 232a. Further, the basket 234 serves as a "strainer" that receives the sponge flowing out of the drinking fluid pipe together with the sponge washing water.

Next, the controller 214 is electrically connected to the power source 213, the light projecting element 221, the light receiving element 222, the display 216, and the transmitter 250. The controller 214 includes, as functional modules, a measuring unit 214a, a correction and calibration unit 214b, a determination unit 214c, and a storage unit 214d as shown in Fig. 3C, and performs operation control on the measuring instrument 210. A temperature sensor 215 may be further electrically connected to the controller 214. Herein, the measuring unit 214a measures the turbidity of the sponge washing water based on the amount of light transmitted by the light projecting element 221 through the sponge washing water and received by the light receiving element 222. The correction and calibration unit 214b makes a correction and calibration in the turbidity measurement operation. The determination unit 214c determines the quality, i.e., good or bad judgment, of the washing work based on the measurement result of the turbidity. The storage unit 214d stores the measurement result and the like. Such a controller 214 is achieved by the use of a microcomputer and includes software corresponding to each function executed by the measuring unit 214a, the correction and calibration unit 214b, and the determination unit 214c, and hardware such as a central processing unit (CPU) and a memory for executing the software. The storage unit 214d is a memory for storing information.

A description will be given of each operation and function of the controller 214.

In the turbidity measurement process, a turbidity of the liquid to be measured and an output signal of the light receiving element 222 (in other words, the amount of light received by the light receiving element 222) are roughly proportional to each other. Thus, as an example, this allows the controller 214 to obtain the measurement result of the turbidity based on correlation information between magnitude of the output signal of the light receiving element 222 and turbidity, the correlation information being obtained and stored in advance. As another example, this allows the controller 214 to determine the quality, i.e., good or bad judgment, of the washing work by comparing measurement result of the turbidity and a washing degree determination reference value obtained and stored in advance.

Further, the light projecting element 221 and the light receiving element 222 have temperature characteristics, so that the controller 214 corrects the measurement result of the turbidity based on an ambient temperature, measured by the temperature sensor 215, at which the measuring instrument 210 is used.

Further, the measurement result varies due to raw water that is a source of the liquid to be measured, or dirt or scratches on the sampling container 230. Therefore, before the measurement, the controller 214 corrects the correlation information (calibration curve) between the magnitude of the output signal of the light receiving element 222 and the turbidity through measuring the raw water filled into a sampling container 230 to be used.

Furthermore, the controller 214 stores, in the storage unit 214d, information such as a data number, obtained measurement result of the turbidity, determination result of the washing degree, whether or not the calibration has been made and a calibration value, the ambient temperature, the measurement date and time, and the like.

The display 216 according to the present embodiment includes a washing work quality indicator 216a, an operation indicator 216b, and a display portion 216c for use in displaying a measured value or the like.

The switches 218 according to the present embodiment include three push switches for power (on/off), measurement, and calibration provided on the front surface 211.

A brief description will be given of the operation of the washing degree determination device 200 configured as described above with reference to Fig. 3E.

In steps S1, S2, the power switch in the switches 218 of the washing degree determination device 200 is pressed to put the washing degree determination device 200 into operation.

Next, in step S3, tap water (raw water) used for the sponge washing is filled into the liquid storage portion 231 of the sampling container 230, the sampling container 230 is set in the holder 212 of the measuring instrument 210, and then the calibration work is conducted. In the calibration work, pressing the calibration button of the switches 218 of the washing degree determination device 200 causes the controller 214 to automatically correct the correlation information (calibration curve) between the magnitude of the output signal of the light receiving element 222 and the turbidity. Note that it is possible to skip S3 and then proceed to the next step S4.

In step S4, the measurement work of the sponge washing water is conducted. Specifically, first, as a preliminary arrangement, with respect to the drinking fluid pipe extending from the outlet of the storage container 10 to the liquid spout 54 of the dispensing device 50 in the liquid supply system 70, the worker drains remaining beer from the drinking fluid pipe, washes the drinking fluid pipe with the tap water already subjected to the calibration work (wash with running water), and blows air into the drinking fluid pipe in this order. Then, after loading the sponge into the supply pipe 30, the worker operates the lever 56 of the spout 54 while applying a predetermined pressure to the sponge with the pressurizing source 15, and collects, that is, makes a sampling of, the sponge washing water and the sponge flowing out of the spout 54 into the sampling container 230 having the basket 234 attached thereto. In this manner, a predetermined amount of the sponge washing water is stored into the liquid storage portion 231, and the sponge is received in the basket 134.

Next, the sampling container 230 in which the sponge washing water has been collected is set into the holder 212 of the measuring instrument 210, and the measurement button of the switches 218 of the washing degree determination device 200 is pressed. This causes the controller 214 to obtain the turbidity of the sponge washing water based on the magnitude of the output signal of the light receiving element 222 using the calibration curve corrected in step S3 and to make a temperature correction with respect to the measurement result based on temperature information obtained from the temperature sensor 215. The measured value thus corrected is displayed on the display portion 216c.

Further, the controller 214 compares the turbidity value thus obtained with the predetermined washing degree determination reference value for being used in determination of the quality, i.e., good or bad judgment, of the washing work within the drinking fluid pipe to determine the quality of the washing work.

Furthermore, the controller 214 stores, in the storage unit 214d, information on the measurement of the turbidity of the sponge washing water (liquid to be measured), such as the measurement result of the turbidity, the determination result of the washing degree, whether or not the calibration has been made and the calibration value, the ambient temperature, the measurement date and time, and the like.

This is the end of the measurement operation (step S4).

In the next step S5, when the worker turns the power switch of the measuring instrument 210 off, or after the elapse of a predetermined time period, the measuring instrument 210 automatically stops operating. This is the end of the turbidity measurement and determination operation of the washing degree made by the washing degree determination device 200.

Next, a description will be given of the transmitter 250.

The transmitter 250 transmits at least the information on the measurement result of the turbidity among the above-described various pieces of information obtained by the controller 214 to the communication unit 140 in the liquid supply system 100 by using, for example, the near-field radio communication function 560. Then, the communication unit 140 transmits the information on the measurement result of the turbidity together with other information including, for example, time information and store information to the management server 300 over the communication line 550.

The transmitter 250 in the washing degree determination device 200 may be configured to directly communicate with the management server 300 over the communication line 550, in this configuration, since the washing degree determination device 200 is a portable device, it is therefore required that at least the store information be input. So, it is more convenient that the washing degree determination device 200 communicates with the liquid supply system 100 installed in each store.

Further, the worker may directly input at least the information on the measurement result of the turbidity among the above-described various pieces of information to the management server 300 without the near-field radio communication function 560 or the communication line 550.

Using the information on the measurement result of the turbidity of the sponge washing water by the washing degree determination device 200 in order to predict the contamination within the drinking fluid pipe extending from the outlet of the storage container 10 to the spout 54 is significant, since the information on the measurement result of the turbidity is acquired directly at a jobsite and is an important factor in correcting the contamination level within the pipe.

As described above, according to the present embodiment, the turbidity measurement is made with respect to the sponge washing water, however the turbidity measurement may be made with respect to washing water having used in washing with tap water. Then, information on the turbidity measurement on the washing water having used for the water washing may be used for correcting the contamination level.

Next, a description will be given of the sales information management system 400. Note that the following description is conditional on a store being equipped with the sales information management system 400.

As described above, the sales information management system 400 is equivalent to a POS system and reads sales information and the like upon, for example, payment made by a customer, and gives, to the management server 300 via the communication line 550, sales information containing pieces of information such as a sales unit capacity of the beverage 20 that is a prescribed capacity per one sale, the number of sales of the beverage 20, and sales time of the beverage 20 that are associated with each other. Here, the aforementioned "prescribed capacity per one sale", that is, the "sales unit capacity" is equivalent to, for example, a capacity of one cup which is determined with respect to the beverage container 40 in each size. For example, it corresponds to a value of 300 ml in the case of a medium beer mug, a value of 500 ml in the case of a large beer mug, and the like. This sales unit capacity can be set by being input from the sales information management system 400 or by being input to the management server 300 when the management server 300 has a functional part, for example, a consumption analysis part, and can be set for each of the plurality of types of beverage containers 40.

The use of such a sales information management system 400 allows, for example, the number of sales of the beverage 20 such as beer to be recorded together with the time information representing date and time. Note that the time when the sales information management system 400 obtains the sales information and the like is not limited to the time of payment, and may be a time of inputting an order to a terminal carried by a store clerk, or the like.

Further, the applicant recognizes that there is a correlation between a staying time (a time during which the beverage 20 does not flow and stays in the drinking fluid pipe) of the beverage 20 in the drinking fluid pipe extending from the outlet of the storage container 10 to the spout 54 and contamination in the pipe, then whether or not the beverage 20 flows through the pipe and how frequent the beverage 20 flows are important factors in predicting contamination in the pipe. That is, via the sales information acquired by the sales information management system 400, information such as the the number of sales and sales trend of the beverage 20 can be obtained in real time, thereby allowing, for example, the staying time of the beverage 20 in the pipe to be grasped in real time. Therefore, using such sales information in order to predict contamination in the drinking fluid pipe extending from the outlet of the storage container 10 to the spout 54 is an important factor in predicting contamination and is thus significant.

As a method other than the use of the sales information in measuring the above-described staying time, when the liquid supply system 100 includes the above-described flow rate sensor, the staying time can also be obtained based on on/off of sensing of the flow rate sensor.

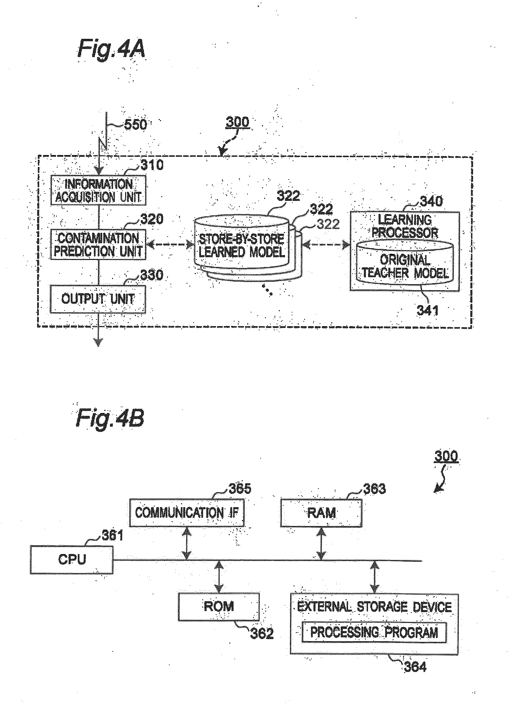

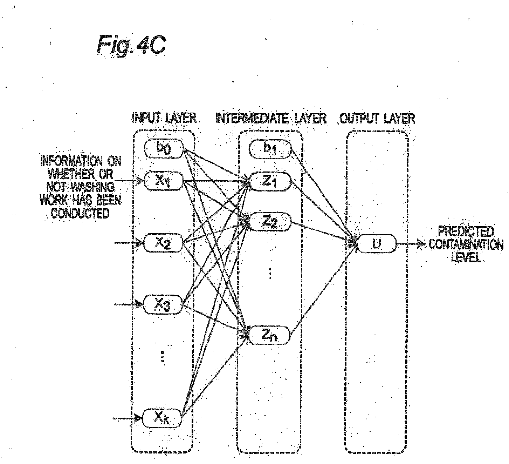

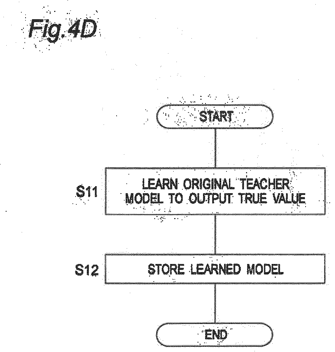

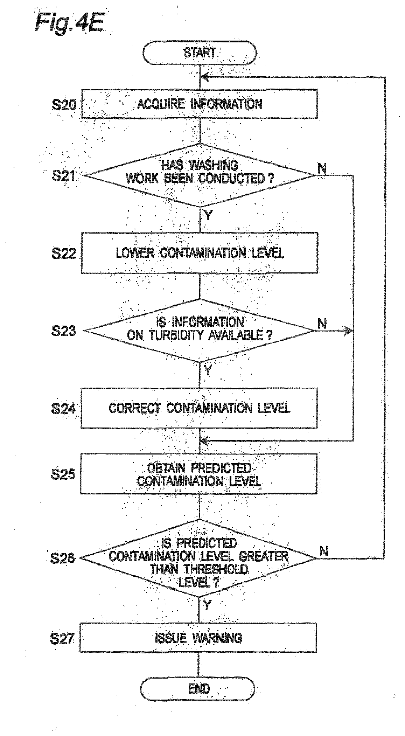

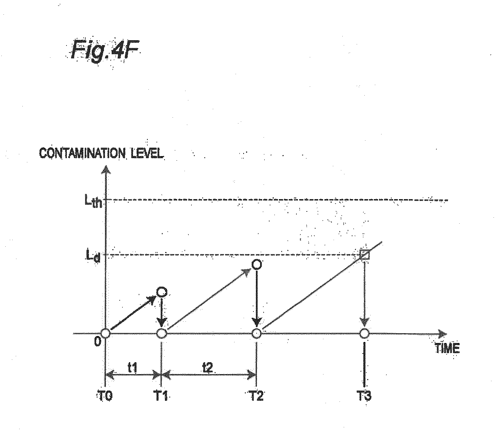

Next, a description will be given of the management server 300 with reference to Figs. 4A to 4E (sometimes collectively referred to as Fig. 4).

The management server 300 includes a machine learning unit and predicts, by machine learning based on at least the above-described various pieces of information given from the liquid supply system 100 over the communication line 550, contamination in the drinking fluid pipe extending from the outlet of the storage container 10 to the spout 54 in the liquid supply system 100 for each store.

Further, the management server 300 predicts the contamination in the pipe for each store by machine learning with at least one of the information on the measurement result of the turbidity from the liquid supply system 100 and the information given from the sales information management system 400 over the communication line 550 added.

As is clear from the above description, the information given to the management server 300 is acquired from each store (restaurant or the like) having the liquid supply system 100. Therefore, the management server 300 is not a server that it collectively predict contamination in the pipes in all stores, but a server that individually predicts contamination in the drinking fluid pipe in the liquid supply system 100 installed in each store.

The operation of the management server 300 is schematically described as follows.

A learning processor 340 trains an original teacher model 341 with various pieces of information acquired from each store to construct a store-by-store learned model 322 unique to each store. Then, a contamination prediction unit 320 predicts, by using the store-by-store learned model 322, contamination in the drinking fluid pipe as described above. The store-by-store learned model 322 is further subjected to the learning processing of the learning processor 340 by using various pieces of information further acquired from each store over time. That is, the store-by-store learned model 322 gradually develops.

Examples of the prediction about contamination as output include a presentation of time information such as the number of days until the contamination level of the drinking fluid pipe exceeds a threshold level (threshold level Lth shown in Fig. 4F), a presentation of a ratio of a current contamination level to the threshold level Lth, and the like.

Alternatively, the management server 300 can construct the store-by-store learned model based on various pieces of information acquired from each store without using the original teacher model. Then, the prediction about contamination can be made by using the store-by-store learned model thus constructed.

The original teacher model is a model representing a relationship between at least the above-described information such as the information on whether or not the washing work has been conducted, the information on the liquid temperature of the beverage 20 and the information on the liquid type of the beverage 20, and the contamination in the drinking fluid pipe. The original teacher model is equivalent to a model constructed under experimental conditions. That is, as for the contamination level in the drinking fluid pipe extending from the outlet of the storage container 10 to the spout 54 in the liquid supply system 100, schematic behavior shown in Fig. 4F is conceivable. Specifically, the contamination level of the drinking fluid pipe begins to rise upon the start of use of the liquid supply system 100 at time T0, and when the drinking fluid pipe is washed after t1 hours, it is conceivable that the contamination level will return to zero or nearly zero. The same applies to a period from time T1 to time T2. Note that it is conceivable that the contamination level that has recovered will gradually rise from zero due to time elapsed. Furthermore, at time T3, in a case that the worker conducts washing and causes the washing degree determination device 200 to measure the turbidity of the washing water, the measurement result, that is, a contamination level Ld of the drinking fluid pipe that is a measured value, in other words, a true value of the contamination level can be obtained. Further, in addition to the washing, when the supply pipe 30 that is a resin tube and is a part of the drinking fluid pipe is replaced, it is conceivable that the contamination level will return to zero or nearly zero.

The relationship between such time information and the like, and the contamination level can be obtained under experimental conditions (at a laboratory), and can be used as the original teacher model.

Further, the above-described information on whether or not the washing work has been conducted can further contain the elapsed time information (equivalent to the above-described t1, t2 hour) representing the period from the previous washing work to the current washing work, as described above.

Based on the above, a description will be given of the management server 300 with reference to Fig. 4A.

Such a management server 300 include, as functional modules, an information acquisition unit 310, the contamination prediction unit 320, an output unit 330, the learning processor 340, and the store-by-store learned model 322 as shown by the block diagram in Fig. 4A.

The information acquisition unit 310 acquires, together with the time information and the store information, two pieces of information, the information on whether or not the washing work has been conducted and the information on the liquid temperature of the beverage 20, that is, the information on the temperature of the beverage 20 stored in the storage container 10, sent by the communication unit 140 of the liquid supply system 100 installed in each store. Further, the information on the liquid type of the beverage 20 input to the management server 300 in advance, the beverage 20 flowing through the liquid supply system 100 installed in each store, is also given to the information acquisition unit 310 for each store.

Further, in each store, when the information on the measurement result of the turbidity of the sponge washing water in the liquid supply system 100 is sent from the washing degree determination device 200 to the liquid supply system 100, the information acquisition unit 310 further acquires the information on the measurement result of the turbidity.

Furthermore, in a case where a store has the sales information management system 400 installed therein, the information acquisition unit 310 acquires, together with other information including the time information and the store information, the above-described sales information sent by the sales information management system 400.

The contamination prediction unit 320 acquires various pieces of information from the information acquisition unit 310 for each store, and uses the above-described store-by-store learned model 322 to predict contamination within the drinking fluid pipe extending from the outlet of the storage container 10 to the spout 54 in the liquid supply system 100.

The output unit 330 acquires the information on the contamination level thus predicted from the contamination prediction unit 320 for each store, and compares the predicted contamination level with the predetermined threshold level Lth (Fig. 4F). Then, the output unit 330 outputs, when the predicted contamination level is greater than the threshold level Lth, an output (warning) to the effect that contamination will occur in the drinking fluid pipe extending from the outlet of the storage container 10 to the spout 54 in the liquid supply system 100.

The learning processor 340 performs the above-described learning processing on the original teacher model 341 so that the prediction of the contamination in the drinking fluid pipe can be made upon the input of at least the above-described three pieces of information, the information on whether or not the washing work has been conducted, the information on the liquid temperature of the beverage 20, and the information on the liquid type of the beverage 20, and operates in the preparatory stage before the system 500 for predicting contamination in a drinking fluid pipe is put into operation.

As described above, the learning processor 340 further performs, using at least the above-described three pieces of information, the learning processing with respect to the learned model 322 learned based on the original teacher model 341.

Further, as described above, when the management server 300 does not have the original teacher model 341, the learning processor 340 has the store-by-store learned model 322 and performs the learning processing on the store-by-store learned model 322 using at least the information on whether or not the washing work has been conducted, the information on the liquid temperature, and the information on the liquid type described above.

Fig. 4D is a flowchart showing an example of the learning processing performed by the learning processor 340.

In step S11, the learning processor 340 performs the learning processing on the above-described original teacher model 341. The original teacher model 341 is a model serving as a base of the learned model 322 constructed for each store and is stored in a storage unit of the learning processor 340. Further, the learning processing performed on the original teacher model 341 is the same as processing performed by known methods. The learning processor 340 adjusts parameters (a weight coefficient, a bias, and the like) by using a known method so as to enable the original teacher model 341 to output a true contamination level. Further, each parameter of the store-by-store learned model 322 is usually store specific.

The learning processor 340 further performs, using various pieces of information further acquired from each store, the learning processing on the store-by-store learned model 322 constructed as described above.

In the next step S12, the learning processor 340 stores, as the learned model 322, the original teacher model 341 subjected to the learning processing, for example, in an external storage device 364 (Fig. 4B).

The learned model 322 is performed the learning processing so as to be able to output a better or satisfactory contamination prediction by performing such learning processing. Then, as described above, the learned model 322 will be constructed with respect to each store, i.e. store-by-store, and the store-by-store learned model 322 will be customized with respect to each store.

Fig. 4B shows an example of a hardware structure of the management server 300. Specifically, the management server 300 is constructed with a computer including, as primary components, a CPU 361, a ROM 362, a RAM 363, the external storage device (for example, flash memory) 364, a communication interface 365, and the like.

Each of the above-described functions of the management server 300 is implemented, for example, by an operation that the CPU 361 reads a processing program, the store-by-store learned model 322, and various pieces of information (such as the information on whether or not the washing work has been conducted, the information on the liquid temperature, and the information on the liquid type), those being stored in the ROM 362, the RAM 363, the external storage device 364 or the like. The RAM 363 serves as, for example, a work area or a temporary save area for data.

Further, a part of or all of the above-described functions may be implemented with processing performed by a DSP instead of or together with the processing performed by the CPU 361. Similarly, a part of or all of each function may be implemented with processing performed by a specifically designed hardware circuit instead of or together with processing performed by software.

As described above, the learned model 322 is performed the learning processing using the original teacher model 341 so as to predict contamination in the drinking fluid pipe upon the input of at least the above-described three pieces of information, the information on whether or not the washing work has been conducted, the information on the liquid temperature of the beverage 20, and the information on the liquid type of the beverage 20.

For such a learned model 322, any learner such as a neural network, a regression tree, a support vector machine (SVM), a Bayesian classifier, or an ensemble model based on an ensemble of such learners can be used. Note that, for the learned model 322, so-called deep learning may be used.

In the present embodiment, as input information having a particularly large impact with respect to computation accuracy of the learned model 322, the information on whether or not the washing work has been conducted, more preferably, information on the frequency of the washing work, that is, information representing time intervals at which the washing work has been conducted, is available. The reason is that it is conceivable that, when the washing work is conducted, the contamination level of the drinking fluid pipe extending from the outlet of the storage container 10 to the spout 54 in the liquid supply system 100 will be lowered or most preferably reset to zero, which greatly affects the prediction about contamination. In addition, the amount of reduction in the contamination level varies in a manner that depends on whether the washing work is the sponge washing or the water washing.

Furthermore, as input information that has a large impact on computation accuracy, the information on the measurement result of the turbidity of the sponge washing water is available. The reason is that the measurement result of the turbidity is information representing the actual contamination level acquired directly at a jobsite and is effective as information based on which the prediction about contamination can be corrected.

As described above, the turbidity measurement may be made with respect to not only on the sponge washing water but also on the washing water having used for water washing with tap water. Then, the information on the turbidity measurement made on the washing water having used for water washing may be used for correcting the contamination level of the pipe.

Further, the learned model 322 (for example, structured data, learned parameter data, and the like) is stored in advance in the external storage device 364 together with the processing program, for example.

Fig. 4C shows an example of the structure of the learned model 322 (herein, a neural network) according to the present embodiment.

The learned model 322 according to the present embodiment has, as an input layer, a plurality of input units for inputting the above-described information such as the information on whether or not the washing work has been conducted. A plurality of input units Xi-Xk are configured to accept the input of the above-described information such as the information on whether or not the washing work has been conducted, the information on the liquid temperature, the information on the liquid type, and further the information on the frequency of the washing work, the information on the turbidity of the sponge washing water, and the sales information.

The other structure of the neural network is the same as provided based on known technologies, and the neural network propagates (performs an operation on) the information input to the input layer in the order of an intermediate layer and an output layer to output the predicted contamination level from the output layer. For example, the intermediate layer includes a plurality of intermediate units Zj (where j is in a range of from 1 to n (the number of units)). Information Xi input to an input unit Xi (where i is in a range of from 1 to k (the number of units)) in the input layer is weighted (accumulated) with each coupling coefficient Wji (not shown), and then input to each intermediate unit Zj in the intermediate layer, and added together in each intermediate unit Zj in the intermediate layer to become a value of each intermediate unit Zj. Further, the value of each intermediate unit Zj in the intermediate layer is subjected to a nonlinear transformation by an input/output function (for example, a sigmoid function), weighted (accumulated) by each coupling coefficient Vj (not shown), and input to and added together in an output unit U in the output layer to become a value of the output unit U in the output layer.

The contamination prediction unit 320 in the management server 300 computes the predicted contamination level through such forward propagation processing in the learned model 322.

Note that the structure of the learned model 322 described above is an example and may be changed in various ways.

As described above, each piece of information given to the information acquisition unit 310 in the management server 300 is store specific, and even for one store, is not uniform and changes. Therefore, with respect to each store, by using the store-by-store learned model 322 unique to each store, causing the contamination prediction unit 320 to predict the contamination level of the drinking fluid pipe allows contamination in the drinking fluid pipe to be managed at a level higher than in the related art and is thus highly effective.

Note that as an example of the structure of the management server 300, the structure including the learning processor 340 has been given above, but the structure of the management server 300 is not limited to the structure including the learning processor 340. That is, another structure may be employed where, a learning device different from the management server 300 performs machine learning with respect to the learned model 322, and the management server 300 acquires the learned model thus learned and stores the learned model in the external storage device 364 or the like.

Further, as an example of the structure of the management server 300, the structure where functions such as the information acquisition unit 310, the contamination prediction unit 320, and the output unit 330 are implemented by a single computer has been given above, but such functions may be implemented by a plurality of computers. Further, programs and data read by such computers may be stored in the plurality of computers in a distributed manner.

A description will be given of the operation of the system 500 for predicting contamination within a drinking fluid pipe having the above-described structure with reference to Fig. 4E.

As described above, at least the information on whether or not the washing work has been conducted and the information on the temperature of the beverage 20 in the storage container 10 together with the time information representing date and time are given from the liquid supply system 100 installed in each store to the management server 300 in the system 500 for predicting contamination. The information on the turbidity of the washing water (having used for either the sponge washing or the water washing) may be further given (step S20). Herein, the information on the liquid type of the beverage 20 is already input to the management server 300.

The management server 300 uses the learned model 322 for each store to predict, based on such pieces of information, contamination within the drinking fluid pipe extending from the outlet of the storage container 10 to the spout 54 in the liquid supply system 100 for each store (step S21 to step S25). Furthermore, when the contamination level of the drinking fluid pipe is greater than the threshold level Lth (Fig. 4F) based on the result of predicting contamination, for each store, the management server 300 outputs information on such a greater contamination level (step S26, step S27).

As described above, since the system 500 for predicting contamination in the present embodiment is configured to output the prediction about contamination in the drinking fluid pipe, controlling or managing the contamination in the drinking fluid pipe can be performed at a level higher than in the related art.

Further, in a case where a store has the sales information management system 400 installed therein, the sales information on the beverage 20 together with the time information can be given from the sales information management system 400 to the management server 300 (step S20). This allows the management server 300 to acquire the information on the staying time of the beverage 20 within the drinking fluid pipe based on the sales information, and additionally use the information on the staying time information to predict contamination.

Therefore, acquiring the sales information allows contamination within the drinking fluid pipe to be controlled or managed at an even higher level.

As described above, according to the present embodiment, the contamination prediction unit 320 in the management server 300 predicts contamination in the drinking fluid pipe of the liquid supply system 100 by using the learned model 322. On the other hand, the contamination prediction unit 320 is not limited to the above-described structure, and with a program, an operation expression, and the like representing the relationship between various pieces of information given to the management server 300 and the prediction about contamination stored in advance in the external storage device 364, the contamination prediction unit 320 may predict, by using the above-described program, contamination in the drinking fluid pipe in accordance with the operation as shown in Fig. 4E.

It is also possible to combine the components shown in the above- . described embodiment. Further, such a combination of the components can exhibit their respective effects.

While the present invention has been fully described in connection with the preferred embodiments with reference to the accompanying drawings, it will be apparent to those skilled in the art that various changes and modifications may be made. Unless such changes and modifications depart from the scope of the present invention as set forth in the accompanying claims, the changes and modifications should be construed as being included within the scope of the present invention.

### INDUSTRIAL APPLICABILITY

The present invention is applicable to a system and method for predicting contamination in a drinking fluid pipe in a liquid supply system.

### DESCRIPTION OF REFERENCE SYMBOLS

- 10: STORAGE CONTAINER
- 20: BEVERAGE
- 50: DISPENSING DEVICE
- 54: SPOUT
- 100: LIQUID SUPPLY SYSTEM
- 110: WASHING MODE DETECTOR
- 121: TEMPERATURE SENSOR
- 131: LIQUID TYPE SENSOR
- 140: COMMUNICATION UNIT
- 200: WASHING DEGREE DETERMINATION DEVICE
- 210: MEASURING INSTRUMENT
- 230: SAMPLING CONTAINER
- 250: TRANSMITTER
- 300: MANAGEMENT SERVER
- 320: CONTAMINATION PREDICTION UNIT
- 322: LEARNED MODEL
- 340: LEARNING PROCESSOR
- 400: SALES INFORMATION MANAGEMENT SYSTEM
- 500: SYSTEM FOR PREDICTING CONTAMINATION
- 550: COMMUNICATION LINE
- 560: NEAR-FIELD RADIO COMMUNICATION FUNCTION

## Claims

1. A system (500) comprising a liquid supply system (100) and a management server (300), wherein the liquid supply system (100) comprises: a dispensing device (50) comprising a spout (54), a drinking fluid pipe which comprises a supply pipe (30), the drinking fluid pipe extending from an outlet of a storage container (10) to the spout (54), the liquid supply system (100) being configured to apply pressure to a beverage in the storage container (10) to supply the beverage to the dispensing device (50) through the supply pipe (30), and cause the beverage to pour into a beverage container (40) through the spout (54), the liquid supply system (100) further comprising a communication unit (140), the liquid supply system (100) being further configured to communicate, to the management server (300) through the communication unit (140), information on whether or not washing work has been conducted on the drinking fluid pipe and information on temperature of the beverage in the storage container (10), and
the management server (300) being configured to output a prediction about contamination in the drinking fluid pipe based on the information on whether or not washing work has been conducted, the information on temperature, and information on liquid type of the beverage,
wherein
the prediction about contamination in the drinking fluid pipe is a number of days until a contamination level of the drinking fluid pipe exceeds a threshold level (Lth), or
the prediction is a ratio of a current contamination level to a threshold level (Lth).

2. The system for predicting contamination in the drinking fluid pipe according to claim 1, wherein the management server (300) includes:
an information acquisition unit (310) configured to receive at least the information on whether or not washing work has been conducted, the information on temperature, and the information on liquid type; and
a contamination prediction unit (320) having a learned model, and configured to input the information received by the information acquisition unit (310) to the learned model to output the prediction about contamination in the drinking fluid pipe.

3. The system for predicting contamination in the drinking fluid pipe according to claim 1 or 2, further comprising a washing degree determination device (200) configured to measure turbidity of washing water having used in washing the drinking fluid pipe extending from the outlet of the storage container (10) to the spout (54), and transmit information on the turbidity to the communication unit (140) or the management server (300) through a transmitter (250), and
the management server (300) configured to additionally use the information on the turbidity and output the prediction about contamination.

4. The system for predicting contamination in the drinking fluid pipe according to claim 3, wherein the management server (300) has a learned model, and in the prediction about contamination by using the learned model, the management server (300) is configured to lower a contamination level based on information representing that washing work has been conducted, and use the information on the turbidity to correct the contamination level.

5. The system (500) for predicting contamination in the drinking fluid pipe according to any one of claims 1 to 4, further comprising a sales information management system (400) configured to communicate, to the management server (300), sales information on the beverage in the liquid supply system (100), wherein
the management server (300) is configured to additionally use the sales information and output the prediction about contamination.

6. A method for predicting contamination within a drinking fluid pipe in a liquid supply system (100), the method being executed by using the liquid supply system (100), a washing degree determination device (200), and a management server (300),
the liquid supply system (100) configured to apply pressure to a beverage in a storage container (10) to supply the beverage to a dispensing device (50) through a supply pipe (30), which is a part of the drinking fluid pipe, and cause the beverage to pour into a beverage container (40) through a spout (54) of the dispensing device (50), and further configured to communicate, to the management server through a communication unit (140) of the liquid supply system (100), information on whether or not washing work has been conducted on the drinking fluid pipe extending from an outlet of the storage container (10) to the spout (54) and information on temperature of the beverage in the storage container (10),
the washing degree determination device (200) configured to measure turbidity of washing water having used in washing the drinking fluid pipe extending from the outlet of the storage container (10) to the spout (54), and transmit information on the turbidity to the communication unit (140) or the management server (300), and
the management server (300) configured to output a prediction about contamination in the drinking fluid pipe extending from the outlet of the storage container (10) to the spout (54) based on the information on whether or not washing work has been conducted, the information on temperature, information on liquid type of the beverage, and the information on the turbidity,
wherein
the prediction about contamination is a number of days until a contamination level of the drinking fluid pipe exceeds a threshold level (Lth), or the prediction is
a ratio of a current contamination level to a threshold level (Lth), and
the management server (300) includes a learned model, and is configured to make the prediction about contamination by using the learned model, in the prediction about contamination by using the learned model, the management server (300) is configured to lower a contamination level based on information representing that washing work has been conducted, and use the information on the turbidity to correct the contamination level.

## Patentansprüche

1. System (500), aufweisend ein Flüssigkeitszufuhrsystem (100) und einen Verwaltungsserver (300), wobei das Flüssigkeitszufuhrsystem (100) aufweist: eine Abgabevorrichtung (50) aufweisend einen Auslauf (54), ein Trinkfluidrohr, welches ein Zufuhrrohr (30) aufweist, wobei sich das Trinkfluidrohr von einem Auslass eines Speicherbehälters (10) zu dem Auslauf (54) erstreckt, wobei das Flüssigkeitszufuhrsystem (100) konfiguriert ist, Druck auf ein Getränk in dem Speicherbehälter (10) aufzubringen, um das Getränk der Abgabevorrichtung (50) durch das Zufuhrrohr (30) zuzuführen, und zu bewirken, dass das Getränk in einen Getränkebehälter (40) durch den Auslauf (54) gefüllt wird, wobei das Flüssigkeitszufuhrsystem (100) ferner eine Kommunikationseinheit (140) aufweist, wobei das Flüssigkeitszufuhrsystem (100) ferner konfiguriert ist, um dem Verwaltungsserver (300) durch die Kommunikationseinheit (140) Informationen darüber, ob Spülarbeit an dem Trinkfluidrohr durchgeführt wurde oder nicht, und Informationen bezüglich der Temperatur des Getränks in dem Speicherbehälter (10) mitzuteilen, und
wobei der Verwaltungsserver (300) konfiguriert ist, eine Vorhersage über Verschmutzung in dem Trinkfluidrohr basierend auf den Informationen darüber, ob Spülarbeit durchgeführt wurde oder nicht, den Informationen bezüglich der Temperatur und Informationen bezüglich eines Flüssigkeitstyps des Getränks auszugeben,
wobei
die Vorhersage über die Verschmutzung in dem Trinkfluidrohr eine Anzahl von Tagen ist, bis ein Verschmutzungsniveau des Trinkfluidrohrs ein Schwellenniveau (Lth) überschreitet, oder
die Vorhersage ein Verhältnis eines aktuellen Verschmutzungsniveaus zu einem Schwellenniveau (Lth) ist.

2. System zum Vorhersagen von Verschmutzung in dem Trinkfluidrohr nach Anspruch 1, wobei der Verwaltungsserver (300) aufweist:
eine Informationserfassungseinheit (310), die konfiguriert ist, um zumindest die Informationen darüber, ob Spülarbeit durchgeführt wurde oder nicht, die Informationen bezüglich der Temperatur und die Informationen bezüglich des Flüssigkeitstyps zu erhalten; und
eine Verschmutzungsvorhersageeinheit (320), die ein erlerntes Modell aufweist und konfiguriert ist, die Informationen, die von der Informationserfassungseinheit (310) erhalten werden, in das erlernte Modell einzugeben, um die Vorhersage über die Verschmutzung in dem Trinkfluidrohr auszugeben.

3. System zum Vorhersagen von Verschmutzung in dem Trinkfluidrohr nach Anspruch 1 oder 2, ferner aufweisend eine Spülgradbestimmungsvorrichtung (200), die konfiguriert ist, um Trübheit von Spülwasser zu messen, das bei dem Spülen des Trinkfluidrohrs verwendet wurde, das sich von dem Auslass des Speicherbehälters (10) zu dem Auslauf (54) erstreckt, und Informationen bezüglich der Trübheit an die Kommunikationseinheit (140) oder den Verwaltungsserver (300) durch einen Sender (250) zu übertragen, und
den Verwaltungsserver (300), der konfiguriert ist, um zusätzlich die Informationen bezüglich der Trübheit zu verwenden und die Vorhersage über die Verschmutzung auszugeben.

4. System zum Vorhersagen der Verschmutzung in dem Trinkfluidrohr nach Anspruch 3, wobei der Verwaltungsserver (300) ein erlerntes Modell aufweist, und bei der Vorhersage über Verschmutzung durch Verwenden des erlernten Modells der Verwaltungsserver (300) konfiguriert ist, ein Verschmutzungsniveau basierend auf Informationen, die darstellen, dass Spülarbeit durchgeführt wurde, zu senken und die Informationen bezüglich der Trübheit zu verwenden, um das Verschmutzungsniveau zu korrigieren.

5. System (500) zum Vorhersagen der Verschmutzung in dem Trinkfluidrohr nach einem der Ansprüche 1 bis 4, ferner aufweisend ein Verkaufsinformationsverwaltungssystem (400), das konfiguriert ist, um dem Verwaltungsserver (300) Verkaufsinformationen bezüglich des Getränks in dem Flüssigkeitszufuhrsystem (100) mitzuteilen, wobei
der Verwaltungsserver (300) konfiguriert ist, um zusätzlich die Verkaufsinformationen zu verwenden und die Vorhersage über die Verschmutzung auszugeben.

6. Verfahren zum Vorhersagen von Verschmutzung innerhalb eines Trinkfluidrohrs in einem Flüssigkeitszufuhrsystem (100), wobei das Verfahren durch Verwenden des Flüssigkeitszufuhrsystems (100), einer Spülgradbestimmungsvorrichtung (200) und eines Verwaltungsservers (300) ausgeführt wird,
wobei das Flüssigkeitszufuhrsystem (100) konfiguriert ist, Druck auf ein Getränk in einem Speicherbehälter (10) aufzubringen, um das Getränk einer Abgabevorrichtung (50) durch ein Zufuhrrohr (30) zuzuführen, welches ein Teil des Trinkfluidrohrs ist, und zu bewirken, dass das Getränk in einen Getränkebehälter (40) durch einen Auslauf (54) der Abgabevorrichtung (50) gefüllt wird, und ferner konfiguriert ist, um dem Verwaltungsserver durch eine Kommunikationseinheit (140) des Flüssigkeitszufuhrsystems (100) Informationen darüber, ob Spülarbeit an dem Trinkfluidrohr durchgeführt wurde oder nicht, das sich von einem Auslass des Speicherbehälters (10) zu dem Auslauf (54) erstreckt, und Informationen bezüglich einer Temperatur des Getränks in dem Speicherbehälter (10) mitzuteilen,
wobei die Spülgradbestimmungsvorrichtung (200) konfiguriert ist, Trübheit des Spülwassers, das bei dem Spülen des Trinkfluidrohrs verwendet wurde, das sich von dem Auslass des Speicherbehälters (10) zu dem Auslauf (54) erstreckt, zu messen und Informationen bezüglich der Trübheit an die Kommunikationseinheit (140) oder den Verwaltungsserver (300) zu übertragen, und
der Verwaltungsserver (300) konfiguriert ist, um eine Vorhersage über die Verschmutzung in dem Trinkfluidrohr, das sich von dem Auslass des Speicherbehälters (10) zu dem Auslauf (54) erstreckt, basierend auf den Informationen darüber, ob Spülarbeit durchgeführt wurde oder nicht, den Informationen bezüglich der Temperatur, Informationen bezüglich des Flüssigkeitstyps des Getränks und den Informationen bezüglich der Trübheit auszugeben,
wobei die Vorhersage über die Verschmutzung eine Anzahl von Tagen, bis ein Verschmutzungsniveau des Trinkfluidrohrs ein Schwellenniveau (Lth) überschreitet, ist oder die Vorhersage ein Verhältnis eines aktuellen Verschmutzungsniveaus zu einem Schwellenniveau (Lth) ist, und
der Verwaltungsserver (300) ein erlerntes Modell aufweist und konfiguriert ist, um die Vorhersage über Verschmutzung durch Verwenden des erlernten Modells zu treffen, bei der Vorhersage über Verschmutzung durch Verwenden des erlernten Modells der Verwaltungsserver (300) konfiguriert ist, ein Verschmutzungsniveau basierend auf Informationen zu senken, die darstellen, dass Spülarbeit durchgeführt wurde, und die Informationen bezüglich der Trübheit zu verwenden, um das Verschmutzungsniveau zu korrigieren.

## Revendications

1. Système (500) comprenant un système de fourniture de liquide (100) et un serveur de gestion (300), dans lequel le système de fourniture de liquide (100) comprend : un dispositif de distribution (50) comprenant un bec (54), un tuyau de fluide potable qui comprend un tuyau de fourniture (30), le tuyau de fluide potable s'étendant à partir d'une sortie d'un récipient de stockage (10) au bec (54), le système de fourniture de liquide (100) étant configuré pour appliquer une pression à une boisson dans le récipient de stockage (10) pour fournir la boisson au dispositif de distribution (50) par le biais du tuyau de fourniture (30), et amener la boisson à se déverser dans un récipient à boisson (40) par le biais du bec (54), le système de fourniture de liquide (100) comprenant en outre une unité de communication (140), le système de fourniture de liquide (100) étant en outre configuré pour communiquer au serveur de gestion (300), par le biais de l'unité de communication (140), des informations indiquant si un travail de lavage a été effectué ou non sur le tuyau de fluide potable et des informations sur la température de la boisson dans le récipient de stockage (10), et
le serveur de gestion (300) étant configuré pour produire une prédiction sur la contamination dans le tuyau de fluide potable sur la base des informations indiquant si un travail de lavage a été effectué ou non, des informations sur la température et des informations sur le type de liquide de la boisson,
dans lequel
la prédiction sur la contamination du tuyau de fluide potable est un nombre de jours jusqu'à ce qu'un niveau de contamination du tuyau de fluide potable dépasse un niveau seuil (Lth), ou la prédiction est un rapport entre un niveau de contamination actuel et un niveau seuil (Lth).

2. Système de prédiction de la contamination dans le tuyau de fluide potable selon la revendication 1, dans lequel le serveur de gestion (300) inclut :
une unité d'acquisition d'informations (310) configurée pour recevoir au moins les informations indiquant si un travail de lavage a été effectué ou non, les informations sur la température et les informations sur le type de liquide ; et
une unité de prédiction de la contamination (320) ayant un modèle appris, et configurée pour introduire les informations reçues par l'unité d'acquisition d'informations (310) dans le modèle appris afin de produire la prédiction sur la contamination dans le tuyau de fluide potable.

3. Système de prédiction de la contamination dans le tuyau de fluide potable selon la revendication 1 ou 2, comprenant en outre un dispositif de détermination du degré de lavage (200) configuré pour mesurer la turbidité de l'eau de lavage utilisée pour laver le tuyau de fluide potable s'étendant à partir de la sortie du récipient de stockage (10) au bec (54), et transmettre des informations sur la turbidité à l'unité de communication (140) ou au serveur de gestion (300) par le biais d'un transmetteur (250), et
le serveur de gestion (300) étant configuré pour utiliser en outre les informations sur la turbidité et produire la prédiction sur la contamination.

4. Système de prédiction de la contamination dans le tuyau de fluide potable selon la revendication 3, dans lequel le serveur de gestion (300) a un modèle appris, et dans la prédiction sur la contamination en utilisant le modèle appris, le serveur de gestion (300) est configuré pour abaisser un niveau de contamination sur la base des informations représentant que le travail de lavage a été effectué, et utiliser les informations sur la turbidité pour corriger le niveau de contamination.

5. Système (500) de prédiction de la contamination dans le tuyau de fluide potable selon l'une quelconque des revendications 1 à 4, comprenant en outre un système de gestion des informations de vente (400) configuré pour communiquer, au serveur de gestion (300), des informations de vente sur la boisson dans le système de fourniture de liquide (100), dans lequel
le serveur de gestion (300) est configuré pour utiliser en outre les informations de vente et produire la prédiction sur la contamination.

6. Procédé de prédiction de la contamination à l'intérieur d'un tuyau de fluide potable dans un système de fourniture de liquide (100), le procédé étant exécuté en utilisant le système de fourniture de liquide (100), un dispositif de détermination du degré de lavage (200), et un serveur de gestion (300),
le système de fourniture de liquide (100) étant configuré pour appliquer une pression à une boisson dans un récipient de stockage (10) afin de fournir la boisson à un dispositif de distribution (50) par le biais d'un tuyau de fourniture (30), qui fait partie du tuyau de fluide potable, et amener la boisson à se déverser dans un récipient à boisson (40) par le biais d'un bec (54) du dispositif de distribution (50), et étant configuré en outre pour communiquer, au serveur de gestion, par l'intermédiaire d'une unité de communication (140) du système de fourniture de liquide (100), des informations indiquant si un travail de lavage a été effectué sur le tuyau de fluide potable s'étendant à partir d'une sortie du récipient de stockage (10) au bec (54) et des informations sur la température de la boisson dans le récipient de stockage (10),
le dispositif de détermination du degré de lavage (200) étant configuré pour mesurer la turbidité de l'eau de lavage utilisée pour laver le tuyau de fluide potable s'étendant à partir de la sortie du récipient de stockage (10) au bec (54), et transmettre des informations sur la turbidité à l'unité de communication (140) ou au serveur de gestion (300), et
le serveur de gestion (300) étant configuré pour produire une prédiction sur la contamination du tuyau de fluide potable s'étendant à partir de la sortie du récipient de stockage (10) au bec (54) sur la base des informations indiquant si un travail de lavage a été effectué, des informations sur la température, des information sur le type de liquide de la boisson et des informations sur la turbidité,
dans lequel
la prédiction sur la contamination est un nombre de jours jusqu'à ce qu'un niveau de contamination du tuyau de fluide potable dépasse un niveau seuil (Lth), ou la prédiction est un rapport entre un niveau de contamination actuel et un niveau seuil (Lth), et
le serveur de gestion (300) inclut un modèle appris, et est configuré pour faire la prédiction sur la contamination en utilisant le modèle appris, dans la prédiction sur la contamination en utilisant le modèle appris, le serveur de gestion (300) est configuré pour abaisser un niveau de contamination sur la base d'informations représentant que le travail de lavage a été effectué, et utiliser les informations sur la turbidité pour corriger le niveau de contamination.
